(19) Europäisches Patentamt
European Patent Office
Office européen des brevets

(11) Veröffentlichungsnummer: **0 297 030 B1**

# (12) EUROPÄISCHE PATENTSCHRIFT

(45) Veröffentlichungstag der Patentschrift: **17.03.93**

(21) Anmeldenummer: **88810319.9**

(22) Anmeldetag: **19.05.88**

(51) Int. Cl.[5]: **C08G 59/04**, C07D 303/24, C07D 319/06, C07D 317/18, C07D 493/10, C08G 2/18, C08G 67/00, C07D 407/12

## (54) Epoxidgruppenhaltige Polycycloacetale.

(30) Priorität: **27.05.87 CH 2050/87**

(43) Veröffentlichungstag der Anmeldung:
**28.12.88 Patentblatt 88/52**

(45) Bekanntmachung des Hinweises auf die Patenterteilung:
**17.03.93 Patentblatt 93/11**

(84) Benannte Vertragsstaaten:
**BE CH DE ES FR GB IT LI NL**

(56) Entgegenhaltungen:
**DE-A- 2 135 382**
**US-A- 2 963 464**
**US-A- 3 884 944**
**US-A- 4 656 294**

**PATENT ABSTRACTS OF JAPAN Band 7, Nr.242 (C-192)(1387), 27.Oct.1983; & JP-A-58 131 985**

(73) Patentinhaber: **CIBA-GEIGY AG**
**Klybeckstrasse 141**
**CH-4002 Basel(CH)**

(72) Erfinder: **Cotting, Jacques-Alain, Dr.**
**Alfons-Aeby-Strasse 19C**
**CH-3186 Düdingen(CH)**
Erfinder: **Renner, Alfred, Dr.**
**Marcoup 2**
**CH-3286 Muntelier(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 297 030 B1

## Beschreibung

Die Erfindung betrifft epoxidgruppenhaltige Polycycloacetale, ein Verfahren zu deren Herstellung und deren Verwendung zur Herstellung von vernetzten ausgehärteten Produkten.

Epoxidharze finden Anwendung in zahlreichen Gebieten, z.B. als Klebstoffe, Lacke, Pressmassen, Isolatoren und Verbundwerkstoffe, und im Handel wird eine Vielfalt chemisch verschiedener Epoxidharze angeboten. Die üblicherweise verwendeten Epoxidharze sind von einem Bisphenol, einer Dicarbonsäure oder von einem Diamin und Epichlorhydrin abgeleitete Glycidylderivate. Für bestimmte Anwendungen werden auch Polyglycidylether von aliphatischen und cycloaliphatischen Polyolen eingesetzt, welche im Vergleich zu den vorerwähnten aromatischen Systemen eine relativ niedrige Viskosität aufweisen und deswegen zum Beispiel für lösungsmittelfreie Beschichtungen geeignet sind.

Epoxidgruppenhaltige cyclische Acetale sind bekannt. So beschreibt die US 3,884,944 cyclische Acetale, die vom 2-Epoxypropoxypivaldehyd abgeleitet sind und bei denen als Diol- bzw. Polyolkomponente z.B. Ethylenglykol, Glycerin, Pentaerythrit, 2,2,6,6-Tetramethylolcyclohexanol oder 2,2,6,6-Tetramethylolcyclohexanon eingesetzt werden. Die US 4,549,008 beschreibt Tetraglycidylether von Tetramethylolcycloalkanolen und Tetramethylolcycloalkanonen, wie z.B. den 2,2,6,6-Tetramethylolcyclohexanol-Tetraglycidylether.

Gegenstand der vorliegenden Anmeldung sind epoxidgruppenhaltige Polycycloacetale der Formel I

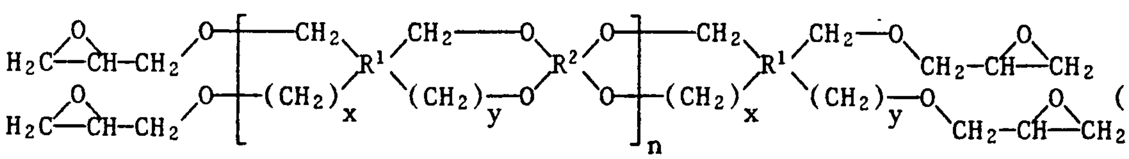

(I),

worin n eine ganze Zahl von 1 bis 500 ist, x und y unabhängig voneinander Null oder 1 sind, $R^1$ für das Kohlenstoffatom oder einen vierwertigen Kohlenwasserstoffrest, dessen Molekulargewicht höchstens 1000 beträgt und welcher noch Ethersauerstoffatome oder Sauerstoffatome in Hydroxyl-, Carbonyl- oder Epoxidgruppen enthalten kann, steht und $R^2$ einen vierwertigen von einem Dialdehyd oder einem Diketon abgeleiteten Kohlenwasserstoffrest bedeutet.

Die erfindungsgemässen Polycycloacetale eignen sich als Epoxidharze zur Herstellung von vernetzten Produkten.

Gegenstand der Erfindung sind somit auch härtbare Gemische enthaltend

(a) ein epoxidgruppenhaltiges Polycycloacetal der Formel I und

(b) einen Härter und/oder einen Härtungskatalysator für Epoxidharze.

Bevorzugt werden Polycycloacetale der Formel I, worin x und y 1 sind, sowie Polycycloacetale der Formel I, worin eines der Symbole x oder y 1 und das andere Symbol Null bedeutet.

Bevorzugt werden auch Polyacetale der Formel I, worin n eine ganze Zahl von 1 bis 50, vorzugsweise von 2 bis 20, ist.

Der Rest $R^1$ der Formel I ist vorzugsweise das Kohlenstoffatom oder ein vierwertiger $C_2$-$C_{24}$-aliphatischer, $C_5$-$C_{24}$-cycloaliphatischer oder $C_6$-$C_{24}$-aromatischer Rest. Die aliphatischen und cycloaliphatischen Reste können Ketogruppen enthalten, durch Hydroxyl- oder Glycidoxygruppen substituiert sein oder auch durch Ethersauerstoffatome in der Kette unterbrochen sein. Die aliphatischen Reste können geradkettig oder verzweigt sein. Die cycloaliphatischen Reste können einen oder mehrere Ringe aufweisen oder können bicyclische oder polycyclische Systeme sein. Die aromatischen Reste können einen oder mehrere, gegebenenfalls kondensierte Ringe aufweisen und umfassen auch vierwertige Reste der Formel

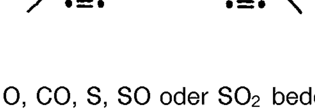

,

worin T Methylen, Isopropyliden, O, CO, S, SO oder $SO_2$ bedeutet.

Besonders bevorzugt sind Polycycloacetale der Formel I, worin $R^1$ das Kohlenstoffatom oder einen vierwertigen $C_2$-$C_{12}$-aliphatischen, $C_5$-$C_{12}$-cycloaliphatischen, oder einen $C_6$-$C_{12}$-aromatischen Rest bedeutet.

Ganz besonders bevorzugt bedeutet $R^1$ das Kohlenstoffatom oder eine Gruppe der Formel

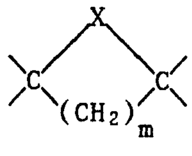 , 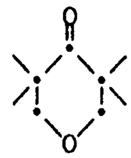 , 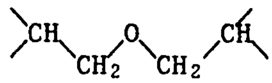 oder

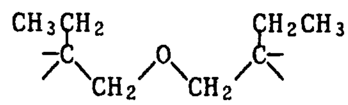

ist, wobei X für die Gruppen

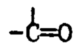, 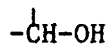 oder 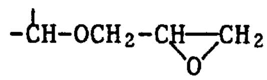

steht und m eine ganze Zahl von 2 bis 5 ist.

$R^1$ hat vorzugsweise ein Molekulargewicht von höchstens 500, besonders bevorzugt von höchstens 250, insbesondere von höchstens 150.

Der Rest $R^2$ der erfindungsgemässen Polycycloacetale ist vorzugsweise ein vierwertiger $C_2$-$C_{12}$-aliphatischer, $C_5$-$C_{12}$-cycloaliphatischer oder $C_6$-$C_{12}$-aromatischer Rest. Bei den aliphatischen, cycloaliphatischen und aromatischen Resten $R^2$ gilt das für die entsprechenden Reste $R^1$ Gesagte.

Besonders bevorzugt sind Polycycloacetale, worin $R^2$ eine Gruppe der Formel

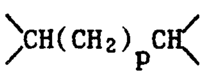 , 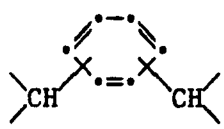 oder 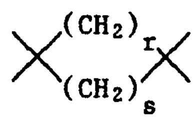

ist,
p Null oder eine ganze Zahl von 1 bis 10 und r und s unabhängig voneinander eine ganze Zahl von 1 bis 5 sind, wobei r + s ≦ 6 ist.

Ganz besonders bevorzugt bedeutet $R^2$ eine Gruppe der Formel

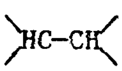 , 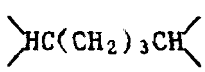 , 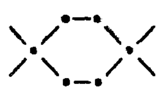 , 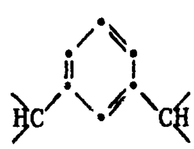 oder

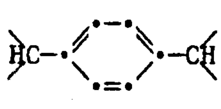 .

Am meisten bevorzugt werden Polycycloacetale, worin $R^2$

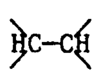 oder 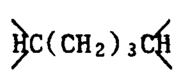

und $R^1$ bei x und y gleich 1

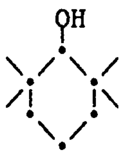 , 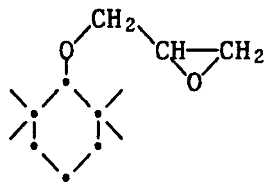 oder 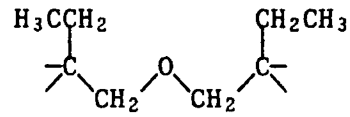

und bei x oder y gleich Null und y oder x gleich 1

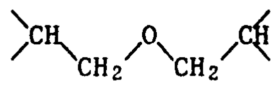

bedeuten.

Die erfindungsgemässen Polycycloacetale kann man z.B. dadurch erhalten, dass man ein Polyol der Formel II

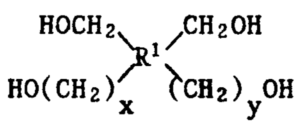 (II)

mit einem Dialdehyd oder einem Diketon zu einem hydroxylgruppenhaltigen Polycyloacetal der Formel III umsetzt

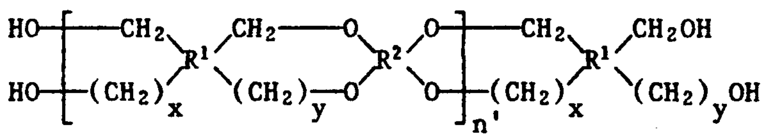 (III)

und anschliessend die Verbindung der Formel III mit Epichlorhydrin glycidyliert, wobei x, y und die Reste $R^1$ und $R^2$ die oben angegebene Bedeutung haben und $n'$ eine ganze Zahl von 1 bis 500 ist.

Hydroxylgruppenhaltige Polycycloacetale sind bekannt und können auf bekannte Weise hergestellt werden. So beschreibt die US 2,963,464 thermoplastische, hochschmelzende Polycycloacetale, die z.B. aus Pentaerythrit und Dialdehyden, wie Glutaraldehyd, oder Diketonen, wie 2,4-Pentandion, hergestellt werden. Aehnliche hydroxylgruppenhaltige Polycycloacetale und deren Herstellung sind auch in den DE-AS 1 247 655 und US 4,374,953 offenbart.

Für die Herstellung der erfindungsgemässen epoxidgruppenhaltigen Polycycloacetale besonders geeignete Polyole der Formel II sind z.B. Pentaerythrit, 2,2,6,6-Tetramethylolcyclohexanol, 2,2,6,6-Tetramethylolcyclohexanon, 2,2,6,6-Tetramethyl-4-oxacyclohexanon, Bis(2,3-dihydroxypropyl)-ether (Diglycerin) und Bis-Trimethylolpropan. Polyole der Formel II sind bekannt und sind zum Teil auch im Handel erhältlich. Tetramethylolcycloalkanole und Tetramethylolcycloalkanone können beispielsweise nach dem in der US 2,462,031 beschriebenen Verfahren hergestellt werden. Bis-Trimethylolpropan und Diglycerin können z.B. durch Kondensation von Trimethylolpropan bzw. Glycerin hergestellt werden. Diese Verbindungen sind auch im Handel erhältlich (z.B. bei der Fa. Perstorp AG, Perstorp, Schweden oder Tokyo Kasei, Japan). Besonders geeignete Dialdehyde oder Diketone sind z.B. Glyoxal, Glutaraldehyd, Succinaldehyd, Terephthalaldehyd, Isophthalaldehyd und 1,4-Cyclohexandion.

Die Polykondensation des Polyols der Formel II mit dem Dialdehyd oder Diketon zum hydroxylgruppenhaltigen Polycycloacetal der Formel III erfolgt vorzugsweise in einem inerten organischen Lösungsmittel, wie z.B. Toluol, mit einem sauren Katalysator, wie z.B. in Anwesenheit von $H_3PO_2$, und unter gleichzeitiger Entfernung von Wasser aus dem Reaktionsgemisch, vorzugsweise durch azeotrope Destillation.

Die Glycidylierung der hydroxylgruppenhaltigen Polycycloacetale der Formel III zu den erfindungsgemässen epoxidgruppenhaltigen Polycycloacetalen der Formel I erfolgt auf an sich bekannte Weise durch

Umsetzung mit Epichlorhydrin in Anwesenheit einer Base, wie z.B. Natronlauge. s gute Resultate werden bei einer Umsetzung mit Epichlorhydrin in Gegenwart eines Phasentransferkatalysators, gefolgt von Dehydrochlorierung des Kondensationsproduktes mit einer Base, z.B. mit Natronlauge, erzielt. Geeignete Phasentransferkatalysatoren sind z.B. tertiäre Sulfoniumsalze, quaternäre Phosphonium-und insbesondere quaternäre Ammoniumsalze. Vorzugsweise können z.B. Tetraethylammonium-, Tetrabutylammonium-, Benzyltrimethylammonium-und insbesondere Tetramethylammoniumsalze, z.B. Chloride, verwendet werden. Bevorzugt wird die Umsetzung mit einem Ueberschuss von Epichlorhydrin ohne Lösungsmittel durchgeführt, wobei der Phasentransferkatalysator und die Base als wässrige Lösungen beigegeben werden. Während der Reaktion können geeigneterweise das Lösungswasser und das bei der Umsetzung entstandene Wasser durch azeotrope Destillation kontinuierlich entfernt werden. Man kann aber auch eine den OH-Gruppen aequivalente Menge Epichlorhydrin in Gegenwart katalytischer Mengen einer Lewis-Säure ($SnCl_4$, $BF_4$ etc.) anlagern und den Poly-2-hydroxy-3-chlorpropylether mit Natronlauge dehydrochlorieren (siehe z.B. US-PS 4,549,008; Bsp. 1).

Die erfindungsgemässen Polycycloacetale der Formel I eignen sich, wie bereits erwähnt, als Epoxidharze zur Herstellung von vernetzten Produkten.

Als Beispiele für Härtungsmittel seien die gebräuchlichen Härtungsmittel für Epoxidharze genannt, einschliesslich der aliphatischen, cycloaliphatischen, aromatischen und heterocyclischen Amine, wie Bis(4-aminophenyl)methan, Anilin-Formaldehyd-Harze, Bis(4-aminophenyl)sulfon, Propan-1,3-diamin, Hexamethylendiamin, Diethylentriamin, Triethylentetramin, 2,2,4-Trimethylhexan-1,6-diamin, m-Xylylendiamin, Bis(4-aminocyclohexyl)methan, 2,2-Bis(4-aminocyclohexyl)propan und 3-Aminomethyl-3,5,5-trimethylcyclohexylamin (Isophorondiamin), Polyaminoamide, wie beispielsweise solche aus aliphatischen Polyaminen und dimerisierten oder trimerisierten Fettsäuren, Polyphenole, wie Resorcin, Hydrochinon, 2,2-Bis(4-hydroxyphenyl)propan und Phenol-Aldehyd-Harze, Polythiole, wie die im Handel unter der Bezeichnung "Thiokole" erhältlichen Polythiole, Polycarbonsäuren und ihre Anhydride, wie zum Beispiel Phthalsäureanhydrid, Tetrahydrophthalsäureanhydrid, Hexahydrophthalsäureanhydrid, Hexachlorendomethylentetrahydrophthalsäureanhydrid, Pyromellitsäuredianhydrid, Benzophenon-3,3,4′,4′-tetracarbonsäuredianhydrid, die Säuren der zuvorgenannten Anhydride sowie auch Isophthalsäure und Terephthalsäure. Geeignete Härtungsmittel sind auch carboxylgruppenendständige Polyester, besonders wenn die erfindungsgemässen härtbaren Gemische für den Oberflächenschutz als Pulverlacke verwendet werden. Es können auch katalytisch wirkende Härtungsmittel verwendet werden, wie beispielsweise Zinnsalze von Alkansäuren (zum Beispiel Zinnoctanoat), Friedel-Crafts-Katalysatoren, wie Bortrifluorid und Bortrichlorid und ihre Komplexe und Chelate, die durch Umsetzung von Bortrifluorid mit zum Beispiel 1,3-Diketonen erhalten werden.

Die Menge des eingesetzten Härtungsmittels richtet sich nach der chemischen Natur des Härtungsmittels und nach den gewünschten Eigenschaften der härtbaren Mischung und des gehärteten Produktes. Die maximale Menge kann leicht ermittelt werden. Wenn das Härtungsmittel ein Amin ist, werden normalerweise 0,75 bis 1,25 Aequivalente Aminwasserstoff pro 1 Epoxidäquivalent eingesetzt. Wenn Polycarbonsäuren oder ihre Anhydride eingesetzt werden, verwendet man gewöhnlich 0,4 bis 1,1 Aequivalente Carboxylgruppe bzw. Anhydridgruppe pro 1 Aequivalent Epoxidgruppe. Bei der Verwendung von Polyphenolen als Härtungsmittel setzt man zweckmässig 0,75 bis 1,25 phenolische Hydroxylgruppen pro 1 Epoxidäquivalent ein.

Katalytisch wirkende Härtungsmittel werden allgemein in Mengen von 1 bis 40 Gewichtsteilen pro 100 Gewichtsteile Epoxidharz eingesetzt.

Man kann bei der Härtung ausserdem Härtungsbeschleuniger einsetzen; solche Beschleuniger sind z.B. tertiäre Amine, deren Salze oder quaternäre Ammoniumverbindungen, z.B. Benzyldimethylamin, 2,4,6-Tris-(dimethylaminomethyl)phenol, 1-Methylimidazol, 2-Ethyl-4-methylimidazol, 4-Aminopyridin, Tripentylammoniumphenolat; oder Alkalimetallalkoholate, wie z.B. Na-Alkoholate von 2,4-Dihydroxy-3-hydroxymethylpentan. Die Härtung der erfindungsgemässen Mischungen wird zweckmässig im Temperaturintervall von 15°C bis 300°C, bevorzugt von 25-250°C, durchgeführt.

Man kann die Härtung in bekannter Weise auch zwei- oder mehrstufig durchführen, wobei die erste Härtungsstufe bei niedriger Temperatur und die Nachhärtung bei höherer Temperatur durchgeführt wird.

Die Härtung kann gewünschtenfalls auch derart in 2 Stufen erfolgen, dass die Härtungsreaktion zunächst vorzeitig abgebrochen bzw. die erste Stufe bei wenig erhöhter Temperatur durchgeführt wird, wobei ein noch schmelzbares und/oder lösliches, härtbares Vorkondensat (sogenannte "B-Stufe") aus der Epoxy-Komponente (a) und dem Härter (b) erhalten wird. Ein derartiges Vorkondensat kann z.B. als Lackstoff und gegebenenfalls zur Herstellung von "Prepregs", Verwendung finden.

Der Ausdruck "Härten", wie er hier gebraucht wird, bedeutet die Umwandlung der löslichen, entweder flüssigen oder schmelzbaren Polyepoxide in feste, unlösliche und unschmelzbare, dreidimensional vernetzte Produkte bzw. Werkstoffe, und zwar in der Regel unter gleichzeitiger Formgebung zu Formkörpern, wie

Giesskörpern, Presskörpern und Schichtstoffen, zu Imprägnierungen, Beschichtungen, Lackfilmen oder Verklebungen.

Die erfindungsgemässen härtbaren Gemische können ferner geeignete Weichmacher, wie Dibutylphthalat, Dioctylphthalat oder Trikresylphthalat, enthalten.

Schliesslich können die erfindungsgemässen härtbaren Gemische vor der Härtung in irgendeiner Phase mit Streck-, Füll- und Verstärkungsmitteln, wie beispielsweise Steinkohlenteer, Bitumen, Textilfasern, Glasfasern, Asbestfasern, Borfasern, Kohlenstoff-Fasern, mineralischen Silikaten, Glimmer, Quarzmehl, Aluminiumoxidhydrat, Bentoniten, Kaolin, Kieselsäureaerogel oder Metallpulvern, z.B. Aluminiumpulver oder Eisenpulver, ferner mit Pigmenten und Farbstoffen, wie Russ, Oxidfarben, Titandioxid u.ä. versetzt werden. Man kann den härtbaren Gemischen ferner auch andere übliche Zusätze, z.B. Flammschutzmittel, wie Antimontrioxid, Thixotropiemittel, Verlaufmittel ("flow control agents"), wie Silicone, Wachse oder Stearate (welche zum Teil auch als Formtrennmittel Anwendung finden), zusetzen.

Die Herstellung der erfindungsgemässen härtbaren Mischungen kann in üblicher Weise mit Hilfe bekannter Mischaggregate (Rührer, Kneter, Walzen etc.) erfolgen.

Die erfindungsgemässen härtbaren Epoxidharzmischungen finden ihren Einsatz vor allem auf den Gebieten des Oberflächenschutzes, der Elektrotechnik, der Laminierverfahren und im Bauwesen. Sie können in jeweils dem speziellen Anwendungszweck angepasster Formulierung, im ungefüllten oder gefüllten Zustand, als Anstrichmitel, Lacke, wie Sinterpulverlacke, als Pressmassen, Tauchharze, Giessharze, Spritzgussformulierungen, Imprägnierharze und Klebmittel, als Werkzeugharze, Laminierharze, Dichtungs- und Spachtelmassen, Bodenbelagsmassen und Bindemittel für mineralische Aggregate verwendet werden.

Die erfindungsgemässen Verbindungen eignen sich besonders für Anwendungen im Oberflächenschutz, wie z.B. bei der Elektrobeschichtung von Metallen, als Lacke und Anstrichstoffe und zudem als Klebstoffe und elektrische Isolierstoffe.

Je nach Struktur des verwendeten Polycycloacetals der Formel I können die erfindungsgemässen Stoffgemische als lösungsmittelfreie flüssige Epoxidharzbeschichtungen, als lösungsmittelhaltige Lacke oder auch als Pulverlacke eingesetzt werden.

Die mit der erfindungsgemässen Verbindung der Formel I hergestellten ausgehärteten Produkte zeichnen sich durch gute chemische, thermische und mechanische Eigenschaften, besonders durch eine hohe Lösungsmittel-, Licht- und Wetterbeständigkeit, aus.

Die folgenden Beispiele erläutern die Erfindung näher.

Beispiel 1: Umsetzungsprodukt von 2,2,6,6-Tetramethylolcyclohexanol und Glutaraldehyd, verethert mit Epichlorhydrin

Teil A: Herstellung des hydroxylgruppenhaltigen Polycycloacetals

90 Teile 2,2,6,6-Tetramethylolcyclohexanol und 0,3 Teile hypophosphorige Säure werden bei 85 °C in 500 Teilen Toluol suspendiert. Im Verlauf von 30 Minuten tropft man 68 Teile einer 50%gen wässrigen Glutaraldehydlösung zu. Man setzt durch Erniedrigung des Druckes auf circa 0,15 bar eine Destillation von Wasser über einen Wasserabscheider in Gang. Nach Ende der Wasserabscheidung wird das Reaktionsgemisch auf 1 bar weitere 2 Stunden unter Rückfluss erhitzt. Man kühlt das Reaktionsgemisch auf 30 °C ab, dekantiert die Toluolphase, löst das entstandene Produkt in 350 Teilen Methanol, destilliert das Methanol am Rotationsverdampfer ab und trocknet bei 120 °C und 0,02 bar.

Ausbeute: 119 Teile (97 % der Theorie)
Erweichungspunkt: 131 °C
$\overline{M}n$ (Gelpermeationschromatographie in THF) = 1247, $\overline{M}w/\overline{M}n$ = 2,078
Hydroxylgehalt: 7,29 Aeq./kg

Teil B: Herstellung des epoxidgruppenhaltigen Polycycloacetals

100 Teile des Polycycloacetals, hergestellt gemäss Teil A, und 2 Teile einer 50%igen wässrigen Lösung von Tetramethylammoniumchlorid werden bei 60 °C in 337 Teilen Epichlorhydrin gelöst. Man setzt durch Erniedrigung des Druckes auf circa 0,15 bar eine Destillation von Epichlorhydrin über einen Wasserabscheider in Gang. Im Verlauf von 1 Stunde tropft man 64 Teile einer 50%igen wässrigen Natronlaugelösung zu, wobei gleichzeitig Wasser durch Destillation ständig entfernt wird. Nach Ende der Wasserabscheidung setzt man die Destillation unter Rückführung von Epichlorhydrin während 5 Stunden fort, kühlt das Reaktionsgemisch auf 30 °C ab, verdünnt mit 220 Teilen Essigester, entfernt das ausgeschiedene Kochsalz durch Filtration und wäscht letzteres mit Essigester nach. Man wäscht die vereinigte Epichlorhydrinlösung mit 100 Teilen einer 10%igen wässrigen $NaH_2PO_4$-Lösung und mit Wasser und trocknet über $Na_2SO_4$. Man destilliert das Epichlorhydrin am Rotationsverdampfer ab und trocknet bei 140 ° C und 0,02 bar.
Ausbeute: 104 Teile (88 % der Theorie)
Erweichungspunkt: 80 ° C
$\overline{M}n$ (Gelpermeationschromatographie in THF) = 2489, $\overline{M}w/\overline{M}n$ = 9,64
Epoxidgehalt: 3,63 Aeq./kg

Beispiel 2: Umsetzungsprodukt von 2,2,6,6-Tetramethylolcyclohexanol und Glyoxal, verethert mit Epichlorhydrin

Teil A: Herstellung des hydroxylgruppenhaltigen Polycycloacetals

Nach dem Verfahren von Beispiel 1, Teil A werden 220 Teile 2,2,6,6-Tetramethylolcyclohexanol in Gegenwart von 0,7 Teilen hypophosphorige Säure in 430 Teilen Toluol suspendiert. Man tropft 72 Teile einer 40%igen wässrigen Glyoxallösung zu.
Ausbeute: 244 Teile (100 % der Theorie)
Erweichungspunkt: 89 ° -94 ° C
$\overline{M}n$ (Gelpermeationschromatographie in THF) = 399, $\overline{M}w/\overline{M}n$ = 1,148
Hydroxylgehalt: 17,16 Aeq./kg

Teil B: Herstellung des epoxidgruppenhaltigen Polycycloacetals

Nach dem Verfahren von Beispiel 1, Teil B werden 140 Teile des Polycycloacetals, hergestellt gemäss Teil A, in Gegenwart von 12 Teilen einer 50%igen wässrigen Lösung von Tetramethylammoniumchlorid in 1110 Teilen Epichlorhydrin gelöst. Im Verlauf von 4 Stunden tropft man 211 Teile einer 50%igen wässrigen Natronlaugelösung zu. Man erhält ein gelbes Oel.
Ausbeute: 100 Teile (48 % der Theorie)
$\overline{M}n$ (Gelpermeationschromatographie in THF) = 746, $\overline{M}w/\overline{M}n$ = 3,095
Epoxidgehalt: 6,11 Aeq./kg

Beispiel 3: Umsetzungsprodukt von Bis-Trimethylolpropan und Glutaraldehyd, verethert mit Epichlorhydrin

Teil A: Herstellung des hydroxylgruppenhaltigen Polycycloacetals

Nach dem Verfahren von Beispiel 1, Teil A werden 125 Teile Bis-Trimethylolpropan (Fa. Perstorp AG, Perstorp, Schweden) in Gegenwart von 0,5 Teilen hypophosphorige Säure in 430 Teilen Toluol suspendiert. Man tropft 91 Teile einer 50%-igen wässrigen Glutaraldehydlösung zu. Man erhält ein hochviskoses Produkt.
Ausbeute: 160 Teile (100 % der Theorie)
$\overline{M}n$ (Gelpermeationschromatographie in THF) = 2233, $\overline{M}w/\overline{M}n$ = 2,33
Hydroxylgehalt: 2,60 Aeq./kg

Teil B: Herstellung des epoxidgruppenhaltigen Polycycloacetals

Nach dem Verfahren von Beispiel 1, Teil B werden 82 Teile des Polycycloacetals, hergestellt gemäss Teil A, in Gegenwart von 0,9 Teilen einer 50%-igen wässrigen Lösung von Tetramethylammoniumchlorid in 100 Teilen Epichlorhydrin gelöst. Im Verlauf von 5 Stunden tropft man 19 Teile einer 50%-igen wässrigen Natronlaugelösung zu. Man erhält ein gelbes Oel.
Ausbeute: 86 Teile (90 % der Theorie)
$\overline{M}n$ (Gelpermeationschromatographie in THF) = 2949, $\overline{M}w/\overline{M}n$ = 3,93
Epoxidgehalt: 0,90 Aeq./kg

Beispiel 4: Umsetzungsprodukt von Bis-Trimethylolpropan und Glyoxal, verethert mit Epichlorhydrin

Teil A: Herstellung des hydroxylgruppenhaltigen Polycycloacetals

Nach dem Verfahren von Beispiel 1, Teil A werden 500 Teile Bis-Trimethylolpropan in Gegenwart von 2,8 Teilen hypophosphorige Säure in 860 Teilen Toluol suspendiert. Man tropft 145 Teile einer 40%-igen wässrigen Glyoxallösung zu. Man erhält ein hochviskoses Produkt.
Ausbeute: 508 Teile (97 % der Theorie)
$\overline{M}n$ (Gelpermeationschromatographie in THF) = 652, $\overline{M}w/\overline{M}n$ = 1,54
Hydroxylgehalt: 12,28 Aeq./kg

Teile B: Herstellung des epoxidgruppenhaltigen Polycycloacetals

Nach dem Verfahren von Beispiel 1, Teil B werden 488 Teile des Polycycloacetals, hergestellt gemäss Teil A, in Gegenwart von 16,3 Teilen einer 50%-igen wässrigen Lösung von Tetramethylammoniumchlorid in 2773 Teilen Epichlorhydrin gelöst. Im Verlauf von 3,5 Stunden tropft man 527 Teile einer 50%-igen wässrigen Natronlaugelösung zu. Man erhält ein braunes Oel.
Ausbeute: 582 Teile (90 % der Theorie)
$\overline{M}n$ (Gelpermeationschromatographie in THF) = 859, $\overline{M}w/\overline{M}n$ = 2,69
Epoxidgehalt: 5,33 Aeq./kg

Beispiel 5: Umsetzungsprodukt von Diglycerin und Glutaraldehyd, verethert mit Epichlorhydrin

Teil A: Herstellung des hydroxylgruppenhaltigen Polycycloacetals

Nach dem Verfahren von Beispiel 1, Teil A werden 415 Teile Diglycerin (Tokyo Kasei über die Fa. Ralupur, Zürich) in Gegenwart von 2,3 Teilen hypophosphorige Säure in 1070 Teilen Toluol suspendiert. Man tropft 333 Teile einer 50%-igen wässrigen Glutaraldehydlösung zu. Man erhält ein hochviskoses Produkt.
Ausbeute: 374 Teile (72 % der Theorie)
$\overline{M}n$ (Gelpermeationschromatographie in THF) = 1020, $\overline{M}w/\overline{M}n$ = 2,15
Hydroxylgehalt: 6,25 Aeq./kg

Teil B: Herstellung des epoxidgruppenhaltigen Polycycloacetals

Nach dem Verfahren von Beispiel 1, Teil B werden 366 Teile des Polycycloacetals, hergestellt gemäss Teil A, in Gegenwart von 7,1 Teilen einer 50%-igen wässrigen Lösung von Tetramethylammoniumchlorid in 1058 Teilen Epichlorhydrin gelöst. Im Verlauf von 3 Stunden tropft man 201 Teile einer 50%-igen wässrigen Natronlaugelösung zu. Man erhält ein gelbes Oel.
Ausbeute: 403 Teile (90 % der Theorie)
$\overline{M}n$ (Gelpermeationschromatographie in THF) = 1798, $\overline{M}w/\overline{M}n$ = 3,07
Epoxidgehalt: 3,90 Aeq./kg

Beispiel 6: Umsetzungsprodukt von Bis-Trimethylolpropan und Glutaraldehyd, verethert mit Epichlorhydrin

Teil A: Herstellung des hydroxylgruppenhaltigen Polycycloacetals

Nach dem Verfahren von Beispiel 1, Teil A werden 125 Teile Bis-Trimethylolpropan in Gegenwart von 0,5 Teilen hypophosphorige Säure in 430 Teilen Toluol suspendiert. Man tropft 67 Teile einer 50%-igen wässrigen Glutaraldehydlösung zu. Man erhält ein hochviskoses Produkt.
Ausbeute: 138 Teile (95 % der Theorie)
$\overline{M}n$ (Gelpermeationschromatographie in THF) = 1155, $\overline{M}w/\overline{M}n$ = 1,76
Hydroxylgehalt: 5,07 Aeq./kg

Teil B: Herstellung des epoxidgruppenhaltigen Polycycloacetals

Nach dem Verfahren von Beispiel 1, Teil B werden 100 Teile des Polycycloacetals, hergestellt gemäss Teil A, in Gegenwart von 0,9 Teilen einer 50%-igen wässrigen Lösung von Tetramethylammoniumchlorid in

235 Teilen Epichlorhydrin gelöst. Im Verlauf von 5 Stunden tropft man 45 Teile einer 50%-igen wässrigen Natronlaugelösung zu. Man erhält ein gelbes Oel.
Ausbeute: 99 Teile (88 % der Theorie)
$\overline{M}n$ (Gelpermeationschromatographie in THF) = 1298, $\overline{M}w/\overline{M}n$ = 1,55
Epoxidgehalt 2,93 Aeq./kg

Anwendungsbeispiele

Beispiel I: Herstellung eines Pulverlackes

Komponenten:

| | |
|---|---|
| 145 g | Epoxidharz gemäss Beispiel 1 |
| 855 g | eines festen carboxylgruppenendständigen gesättigten Polyesters (URALAC® 3400 der Firma Scado, Säuregehalt: 0,61 Aeq./kg) |
| 20 g | eines Gemisches aus 12,5 Gew.t. Alkyl-trimethylammoniumbromid (Morpan® CHSA der Firma ABM Chemicals) und 87,5 Gew.t. eines festen, gesättigten, carboxylgruppenhaltigen Polyesterharzes (Neoxil TPC 83 der Firma Savid) |

Die Komponenten werden gemeinsam während 30 Sekunden in einer Analysenmühle gemahlen. Danach wird das Pulver auf ein gereinigtes Aluminiumblech aufgetragen und 30 Minuten bei 180°C gehärtet. Der entstandene farblose Lackfilm hat eine Dicke von 40-60 µm, und weist sehr gute mechanische Eigenschaften und eine sehr gute Bewitterungsbestädigkeit auf. Die Messwerte sind in der Tabelle angegeben.

Beispiel II: Herstellung eines lösungsmittelhaltigen Lackes

Komponenten:

| | |
|---|---|
| 90 g | Epoxidharz gemäss Beispiel 2 |
| 910 g | eines festen carboxylgruppenendständigen gesättigten Polyesters (URALAC® 3400 der Firma Scado, Säuregehalt: 0,61 Aeq./kg) |
| 20 g | eines Gemisches aus 12,5 Gew.t. Alkyl-trimethylammoniumbromid (Morpan® CHSA der Firma ABM Chemicals) und 87,5 Gew.t. eines festen, gesättigten, carboxylgruppenhaltigen Polyesterharzes (Neoxil TPC 83 der Firma Savid) |
| 680 g | Dimethylformamid |

Die Komponenten werden in DMF gelöst. Danach wird die Lösung auf ein gereinigtes Aluminiumblech aufgetragen und 30 Minuten bei 180°C gehärtet. Der entstandene farblose Lackfilm hat eine Dicke von 50µm und weist sehr gute mechanische Eigenschaften und eine sehr gute Bewitterungsbeständigkeit auf. Die Messwerte sind in der Tabelle angegeben.

Tabelle 1

| Prüfung | Beispiel I | Beispiel II |
|---|---|---|
| Erichsentiefung (DIN 53 156, mm) | > 10 | > 10 |
| Schlagrevers[1] (cm•kg) | >180 | >180 |
| Acetontest[2] (Note) | 2-3 | 3 |
| Haftung[3] (Gitterschnitt, Note) | 0 | 0 |

[1] Auf die beschichteten Aluminiumbleche wird von hinten aus bestimmter Höhe ein Stempel mit einem bekannten Gewicht fallengelassen. Der erhaltene Wert (Höhe mal Gewicht) zeigt den grössten Schlag an, bei dem die Beschichtung noch unbeschädigt bleibt.

[2] Ein mit Aceton getränkter Lappen wird eine Minute lang auf der beschichteten Oberfläche liegen gelassen. Anschliessend wird die behandelte Oberfläche mit dem Fingernagel auf ihre Lösungsmittelresistenz hin überprüft. Die Bewertung erfolgt nach einer Skala von 0 bis 5, wobei 0 ausgezeichnete und 5 schlechte Resistenz bedeutet.

[3] Die Bewertung erfolgt nach einer Skala von 0 bis 5, wobei 0 ausgezeichnete und 5 schlechte Haftung bedeutet.

Beispiel III: Herstellung von Formkörpern

95 Teile Tetraglycidyläther gmeäss Beispiel 6 und 37 Teile Hexahydrophthalsäureanhydrid werden gemischt, in Formen von 150 x 150 x 4 $mm^3$ gegossen und 6 Stunden bei 160 °C und 12 Stunden bei 180 °C gehärtet. Man erhält dunkelgelbe, zähharte, einwandfreie Platten, die zu Prüfstäben zerschnitten werden. Folgende mechanische Eigenschaften werden danach bestimmt:

| | |
|---|---|
| Biegefestigkeit (ISO 178): | 94,5 $N/mm^2$ |
| Randfaserdehnung (ISO 178): | 4,6 % |
| Schlagbiegezähigkeit (ISO 179): | 76,0 $kJ/m^2$ |
| Formbeständigkeit in der Wärme (ISO 75): | 51 °C |
| Kochwasseraufnahme (1 h bei 100 °C): | 0,89 % |

Beispiel IV und V: Herstellung lösungsmittelhaltiger Lacke

| Komponenten (g) | IV | V |
|---|---|---|
| Epoxidharz gemäss Beispiel 4 | 100 | |
| Epoxidharz gemäss Beispiel 5 | | 100 |
| Titandioxid RN 56 (Kronos Titangesellschaft) | 266 | 215 |
| Isobutyl-methylketon | 50 | 40 |
| Carboxylgruppenendständiger gesättigter Polyester (Degolan® VP WL 329 der Fa. DEGUSSA, Säuregehalt: 1,47 Aeq./kg) | 500 | 370 |
| Härtungsbeschleuniger aus 25 Teilen 4-Dimethylamino-pyridin, 25 Teilen Zinkoctoat, 25 Teilen Xylol und 25 Teilen Butanol | 2 | 2 |

Die Lösungen werden jeweils auf ein gereinigtes Aluminiumblech aufgetragen und 20 min bei 200 °C gehärtet. Die Lackfilme haben eine Dicke von ca. 40 µm und weisen die in Tabelle 2 angegebenen Eigenschaften auf. Die Prüfungen werden wie bei den Anwendungsbeispielen I und II beschrieben durchgeführt.

Tabelle 2

| Prüfung | Beispiel IV | Beispiel V |
|---|---|---|
| Erichsentiefung (mm) | 6,8 | 7,6 |
| Schlagrevers (cm•kg) | > 90 | > 90 |
| Acetontest (Note) | 2 | 3 |
| Haftung (Gitterschnitt, Note) | 0 | 0 |
| Dornbiegetest 3 m (DIN 53152) | 180 | 180 |

## Patentansprüche

**1.** Epoxidgruppenhaltige Polycycloacetale der Formel I

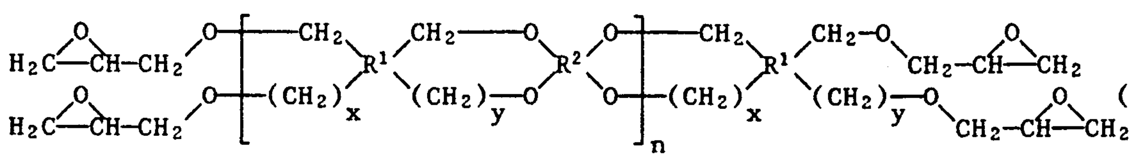

(I),

worin n eine ganze Zahl von 1 bis 500 ist, x und y unabhängig voneinander Null oder 1 sind, $R^1$ für das Kohlenstoffatom oder einen vierwertigen Kohlenwasserstoffrest, dessen Molekulargewicht höchstens 1000 beträgt und welcher noch Ethersauerstoffatome oder Sauerstoffatome in Hydroxyl-, Carbonyl- oder Epoxidgruppen enthalten kann, steht und $R^2$ einen vierwertigen von einem Dialdehyd oder einem Diketon abgeleiteten Kohlenwasserstoffrest bedeutet.

**2.** Polycycloacetale nach Anspruch 1, worin x und y je 1 sind.

**3.** Polycycloacetale nach Anspruch 1, worin eines der Symbole x oder y 1 und das andere Symbol Null bedeutet.

**4.** Polycycloacetale nach Anspruch 1, worin n eine ganze Zahl von 1 bis 50 ist.

**5.** Polyacetale nach Anspruch 1, worin $R^1$ das Kohlenstoffatom oder einen vierwertigen $C_2$-$C_{24}$-aliphatischen, $C_5$-$C_{24}$-cycloaliphatischen oder $C_6$-$C_{24}$-aromatischen Rest bedeutet.

**6.** Polyacetale nach Anspruch 5, worin $R^1$ das Kohlenstoffatom oder einen vierwertigen $C_2$-$C_{12}$-aliphatischen, $C_5$-$C_{12}$-cycloaliphatischen oder $C_6$-$C_{12}$-aromatischen Rest bedeutet.

**7.** Polycycloacetale nach Anspruch 6, worin $R^1$ das Kohlenstoffatom oder eine Gruppe der Formel

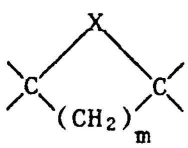 , 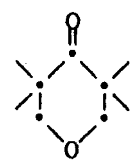 , 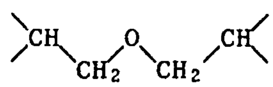 oder

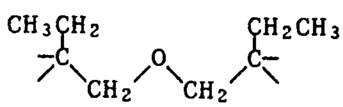

ist, wobei X für die Gruppen

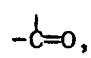, 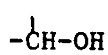 oder 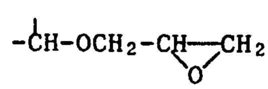

steht und m eine ganze Zahl von 2 bis 5 ist.

**8.** Polycycloacetale nach Anspruch 1, worin $R^2$ einen vierwertigen $C_2$-$C_{12}$-aliphatischen, $C_5$-$C_{12}$-cycloaliphatischen oder $C_6$-$C_{12}$-aromatischen Rest bedeutet.

**9.** Polycycloacetale nach Anspruch 8, worin $R^2$ eine Gruppe der Formel

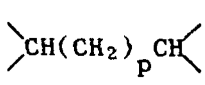 , 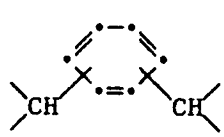 oder 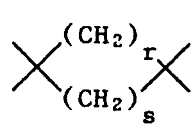

p Null oder eine ganze Zahl von 1 bis 10 und r und s unabhängig voneinander eine ganze Zahl von 1 bis 5 sind, wobei r + s≦ 6 ist.

**10.** Polycycloacetale nach Anspruch 9, worin $R^2$ eine Gruppe der Formel

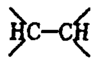 , 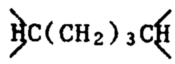 , 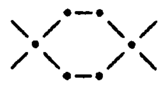 , 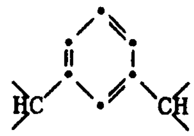 oder

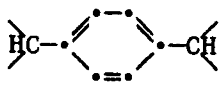 ist.

**11.** Polycycloacetale nach Anspruch 1, worin $R^2$

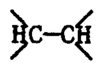 oder 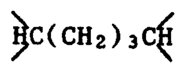

und $R^1$ bei x und y gleich 1

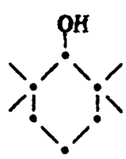 , 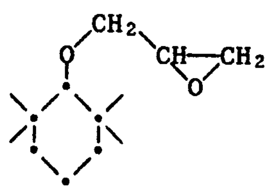 oder 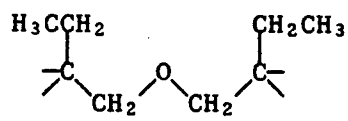

und bei x oder y gleich Null und y oder x gleich 1

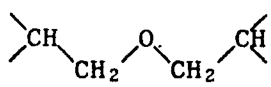

bedeuten.

**12.** Verfahren zur Herstellung von Polycycloacetalen nach Anspruch 1, durch Umsetzung eines Polyols der Formel II

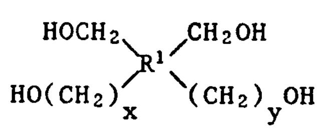 (II)

mit einem Dialdehyd oder einem Diketon zu einem hydroxylgruppenhaltigen Polycycloacetal der Formel III

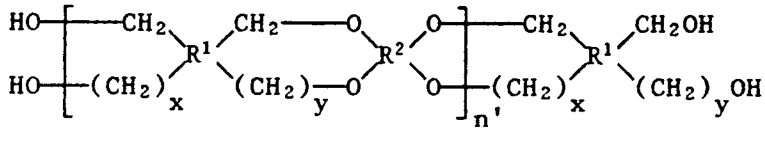 (III)

und anschliessende Glycidylierung der Verbindung der Formel III mit Epichlorhydrin, wobei x, y und die

Reste $R^1$ und $R^2$ die im Anspruch 1 angegebene Bedeutung haben und $n'$ eine ganze Zahl von 1 bis 500 ist.

**13.** Härtbare Gemische enthaltend
(a) ein epoxidgruppenhaltiges Polycycloacetal nach Anspruch 1 und
(b) einen Härter und/oder einen Härtungskatalysator für Epoxidharze.

## Claims

**1.** An epoxide group-containing polycycloacetal of formula I

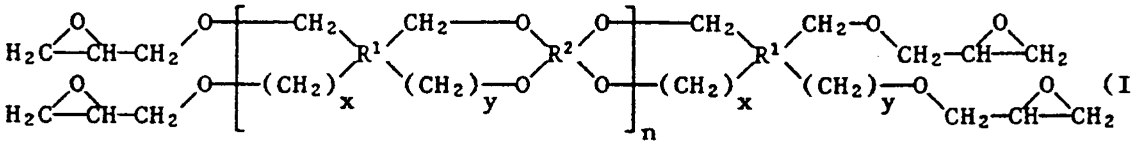 (I),

in which n is an integer from 1 to 500, x and y are each independently of the other 0 or 1, R' is a carbon atom or a tetravalent hydrocarbon radical having a molecular weight not greater than 1000 and which can additionally contain ether oxygen atoms or oxygen atoms in hydroxyl, carbonyl or epoxy groups, and $R^2$ is a tetravalent hydrocarbon radical derived from a dialdehyde or a diketone.

**2.** A polycycloacetal according to claim 1, in which x and y are each 1.

**3.** A polycycloacetal according to claim 1, in which one of the symbols x and y is 1 and the other symbol is zero.

**4.** A polycycloacetal according to claim 1, in which n is an integer from 1 to 50.

**5.** A polycycloacetal according to claim 1, in which $R^1$ is a carbon atom or a tetravalent $C_2$-$C_{24}$aliphatic, $C_5$-$C_{24}$cycloaliphatic or $C_6$-$C_{24}$aromatic radical.

**6.** A polycycloacetal according to claim 5, in which $R^1$ is a carbon atom or a tetravalent $C_2$-$C_{12}$aliphatic, $C_5$-$C_{12}$cycloaliphatic radical or a $C_6$-$C_{12}$aromatic radical.

**7.** A polycycloacetal according to claim 6, in which $R^1$ is a carbon atom or a group of formula

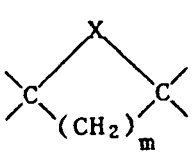 , 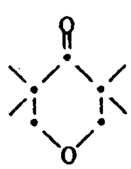 , 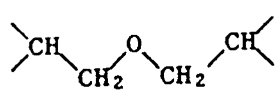 or

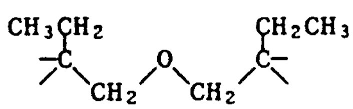

where X is a group selected from

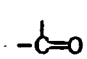, 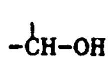 oder 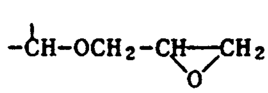

and m is an integer from 2 to 5.

8. A polycycloacetal according to claim 1, in which $R^2$ is a tetravalent $C_2$-$C_{12}$aliphatic radical, $C_5$-$C_{12}$cycloaliphatic radical or $C_6$-$C_{12}$aromatic radical.

9. A polycycloacetal according to claim 8, in which $R^2$ is a group of the formula

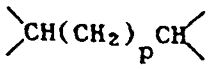 , 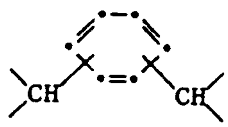 or 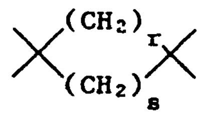

p is zero or an integer from 1 to 10 and r and s are each independently of the other an integer from 1 to 5, where r + s is ≦ 6.

10. A polycycloacetal according to claim 9, in which $R^2$ is a group of formula

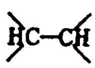 , 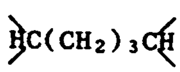 , 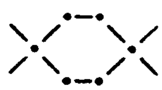 , 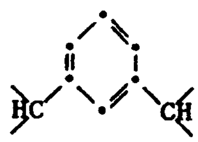 or

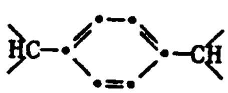

11. A polycycloacetal according to claim 1, in which $R^2$ is

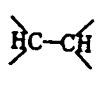 or 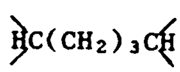

and $R^1$, where x and y are 1, is

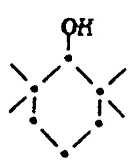 , 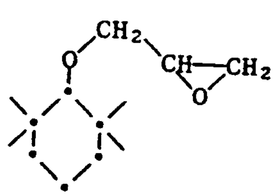 or 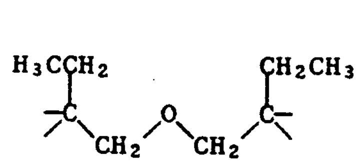

and, where x or y is zero and y or x is 1, is

12. A process for the preparation of a polycycloacetal according to claim 1, which comprises reacting a polyol of formula II

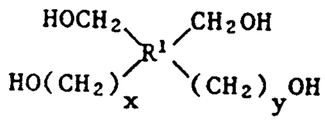

(II)

with a dialdehyde or a diketone to give a hydroxyl group-containing polycycloacetal of formula III

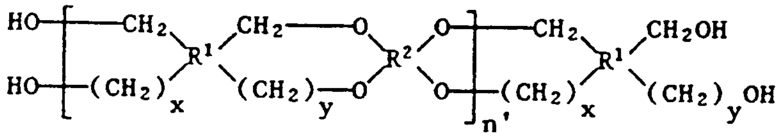

(III)

and subsequently glycidylating the compound of formula III with epichlorohydrin, where x, y and the radicals $R^1$ and $R^2$ are as defined in claim 1 and n' is an integer from 1 to 500.

**13.** A curable mixture comprising
(a) an epoxide group-containing polycycloacetal according to claim 1, and
(b) a hardener and/or a curing catalyst for epoxy resins.

**Revendications**

**1.** Polycycloacétals contenant des groupes époxyde, répondant à la formule I

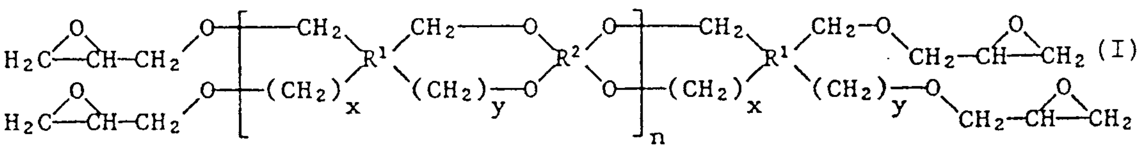

(I)

dans laquelle n est un nombre entier de 1 à 500, x et y sont indépendamment l'un de l'autre 0 ou 1, $R^1$ désigne l'atome de carbone ou un radical hydrocarboné tétravalent dont la masse moléculaire est de 1000 au plus, et qui peut contenir encore des atomes d'oxygène d'éther ou des atomes d'oxygène dans de groupes hydroxyle, carbonyle ou époxyde, et $R^2$ désigne un radical hydrocarboné tétravalent obtenu à partir d'un dialdéhyde ou d'une dicétone.

**2.** Polycycloacétals selon la revendication 1, dans lesquels x et y sont chacun égaux à 1.

**3.** Polycycloacétals selon la revendication 1, dans lesquels un des symboles x ou y est 1 et l'autre symbole est 0.

**4.** Polycycloacétals selon la revendication 1, dans lesquels n est un nombre entier de 1 à 50.

**5.** Polycycloacétals selon la revendication 1, dans lesquels $R^1$ désigne l'atome de carbone ou un radical tétravalent aliphatique en $C_2$-$C_{24}$, cycloaliphatique en $C_5$-$C_{24}$ ou aromatique en $C_6$-$C_{24}$.

**6.** Polycycloacétals selon la revendication 5, dans lesquels $R^1$ désigne l'atome de carbone ou un radical tétravalent aliphatique en $C_2$-$C_{12}$, cycloaliphatique en $C_5$-$C_{12}$ ou aromatique en $C_6$-$C_{12}$.

**7.** Polycycloacétals selon la revendication 6, dans lesquels $R^1$ est l'atome de carbone ou un groupe répondant à la formule

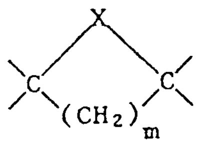 , 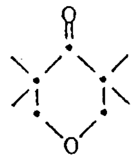 , 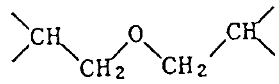 ou

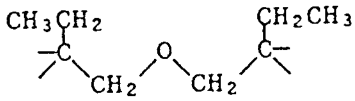

X représentant les groupes

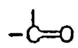 , 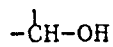 ou 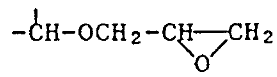

et m un nombre entier de 2 à 5.

**8.** Polycycloacétals selon la revendication 1, dans lesquels $R^2$ désigne un radical tétravalent aliphatique en $C_2$-$C_{12}$, cycloaliphatique en $C_5$-$C_{12}$ ou aromatique en $C_6$-$C_{12}$.

**9.** Polycycloacétals selon la revendication 8, dans lesquels $R^2$ est un groupe répondant aux formules

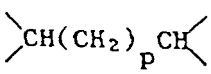 , 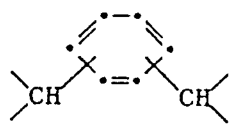 ou 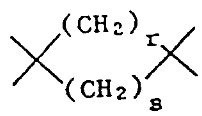

p est 0 ou un nombre entier de 1 à 10, et r et s sont indépendamment l'un de l'autre un nombre entier de 1 à 5, r + s étant inférieur ou égal à 6.

**10.** Polycycloacétals selon la revendication 9, dans lesquels $R^2$ est un groupe répondant aux formules

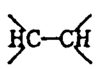 , 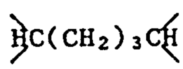 , 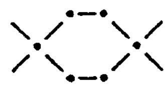 , 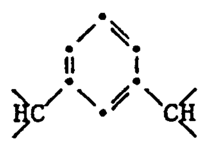 ou

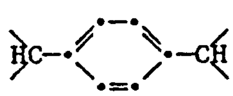

**11.** Polycycloacétals selon la revendication 1, dans lesquels $R^2$ désigne
HC-CH ou $HC(CH_2)_3CH$ et $R^1$, dans le cas où x et y sont égaux à 1, désigne

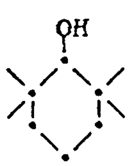 , 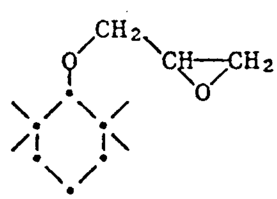 ou 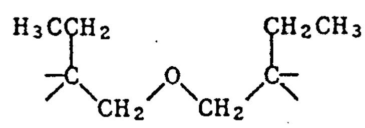

et dans le cas où x ou y est égal à 0 et y ou x est égal à 1, un groupe

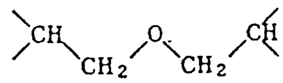

12. Procédé de préparation de polycycloacétals selon la revendication 1, par réaction d'un polyol répondant à la formule II

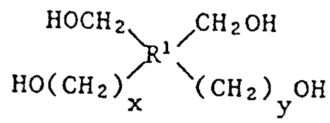

(II)

avec un dialdéhyde ou une dicétone pour obtenir un polycycloacétal contenant des groupes hydroxyle répondant à la formule III

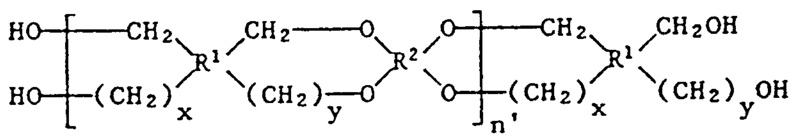

(III)

puis glycidylation du composé répondant à la formule III avec l'épichlorhydrine, x, y et les radicaux $R^1$ et $R^2$ ayant les significations indiquées dans la revendication 1 et n' étant un nombre entier de 1 à 500.

13. Mélanges durcissables contenant
(a) un polycycloacétal contenant des groupes époxyde selon la revendication 1 et
(b) un durcisseur et/ou un catalyseur de durcissement pour résines époxy.